# EUROPÄISCHE PATENTANMELDUNG

(11) **EP 0 716 061 A1**
(43) Veröffentlichungstag der Anmeldung: **12.06.1996**
(21) Anmeldenummer: 95118341.7
(22) Anmeldetag: 22.11.1995
(51) Int. Cl.: C07B 31/00, C07F 9/50, C07C 209/32

(54) **Verfahren zur reduktiven Deoxigenierung mittels eines Redoxkatalysators**

(30) Priorität: 06.12.1994 DE 4443360
(71) Anmelder: BASF AKTIENGESELLSCHAFT, D-67056 Ludwigshafen (DE)
(72) Erfinder: Hagemeyer, Alfred, Dr., D-67063 Ludwigshafen (DE); Rieker, Christopher William, Dr., D-68163 Mannheim (DE); Lautensack, Thomas, D-67317 Altleiningen (DE); Hermeling, Dieter, Dr., D-67459 Böhl-Iggelheim (DE)

(57) **Zusammenfassung**

Verfahren zur Deoxigenierung von organischen Oxo-Verbindungen vor allem Phosphinoxiden zu Phosphinen und Nitrobenzol zu Anilin mit reduzierten Redoxkatalysatoren bei instationärer Reaktionsführung mit entkoppelter reduktiver Regeneration des Redoxkatalysators, wobei der Redoxkatalysator auf eine niedrigere Oxidationsstufe reduziert und wieder zur Deoxigenierung eingesetzt wird.

## Beschreibung

Die Erfindung betrifft ein verfahren zur heterogenkatalytischen reduktiven Deoxigenierung von organischen Verbindungen, die eine schwer reduzierbare Oxogruppe enthalten insbesondere von Phosphinoxiden zu den entsprechenden Phosphinen sowie von Nitrobenzol zu Anilin.

Deoxigenierungen von Oxogruppen enthaltenden organischen Verbindungen stellen in der organischen Chemie eine wichtige Reaktionsklasse für die Synthese oder die Regenerierung von oxidierten Reagentien dar.

Beispiele sind die Reduktion von Phosphinoxiden zu den entsprechenden Phosphinen, die Deoxigenierung von Estern zu Ethern, die Reduktion von aliphatischen und aromatischen Nitroverbindungen zu den Nitrosoverbindungen, Oximen oder Aminen, insbesondere die Reduktion von Nitrobenzol zu Anilin und die Reduktion von Sulfonsäuren zu Mercaptanen.

Ein besonderes technisches Problem stellt die Reduktion von Triphenylphosphinoxid (TPPO) zu Triphenylphosphin (TPP) dar. TPP wird häufig in der organischen Chemie bei Wittigreaktionen zur C=C-Doppelbindungsverknüpfung (z.B. bei der Vitamin-A-Synthese) eingesetzt, wobei TPPO als Reaktionsprodukt oder genauer als Abfallprodukt anfällt und meist noch verbrannt wird. Durch Reduktion von TPPO zu TPP und dessen Wiederverwendung werden dagegen Stoffkreisläufe geschlossen, Abfallmengen reduziert und Verbrennungskapazitäten eingespart.

Gleiches gilt für die Verwendung von TPP als Cokatalysator bei Hydroformylierungen und die Rückgewinnung von Tricyclohexylphosphin aus Tricyclohexylphosphinoxid.

Die Direktreduktion von TPPD zu TPP, etwa mit H₂ oder CO, d.h. die Anwendung der konventionellen stationären Reaktionsführung mit den gängigen und verfügbaren Reduktionsmitteln, gelingt oft nicht pzw. liefert nur geringe Umsätze, da TPPO thermodynamisch wesentlich stabiler ist als TPP und das chemische Gleichgewicht daher weit auf der Seite des TPPO liegt. Die TPPO-Reduktion mit metallischen Reduktionsmitteln kann daher (bei stationärer Reaktionsführung) nur mit sehr oxophilen Metallen (z.B. metallischem Al) durchgeführt werden, die dabei aber zu minderwertigen Salzen (z.B. Oxide oder Halogenide) umgesetzt werden. Diese sind aber so stabil, daß deren Recycling zu den Metallen nur mit erheblichem Aufwand durchführbar ist.

Die stationär geführte Reduktion von Phosphinoxiden über das Dihalogenid mit starken Reduktionsmitteln ist mehrfach beschrieben. So wird gemäß US 3 780 111 das Phosphinoxid zuerst in das Dihalogenid umgewandelt, das anschließend mit Fe reduziert wird. In EP 548 682 wird die Reduktion von Phosphinoxiden oder von (daraus z.B. durch Phosgenierung erhaltenen) Phosphindihalogeniden mit Si oder Si-Legierungen beschrieben. Die Dehalogenierung von TPPCl₂ mit Na ist in DOS 2 638 720 und diejenige mit weißem Phosphor in G. Wunsch et al., Allgem. Chem. 369 (1969) 33 dargelegt. Die katalytische Flüssigphasenhydrierung von TPPCl₂ mit H₂ ist Gegenstand von EP 005 747, wobei Pt, Pd, Rh, Ru und/oder Ir-Katalysatoren zur Anwendung kommen. US 4 727 193 behandelt die Flüssigphasenreduktion von Phosphinoxiden oder -dihalogeniden mit einem Kohlenwasserstoff in Gegenwart von aktivem Kohlenstoff bei erhöhten Temperaturen. Ein Reaktionsmedium bestehend aus Weißöl, Kohlenstoffpulver und TPPO lieferte bei 350°C nach 6 h Reaktionszeit 52 % Umsatz und 68 % Selektivität zu TPP. Dem Vorteil der billigen Reagentien steht hier der Nachteil der geringen Raum-Zeit-Ausbeute bei langen Reaktionszeiten gegenüber; außerdem ergibt sich bei Flüsigphasenreaktionen im Gegensatz zu Gasphasenreaktionen immer das Problem der Abtrennung des Katalysators und/oder des Produktes (z.B. durch Destillation, Extraktion etc.), so daß eine aufwendigere Aufarbeitung erforderlich ist.

Es bestand daher die Aufgabe ein Verfahren vorzuschlagen, das es erlaubt, mit Hilfe von regenerierbaren Reagentien bzw. Katalysatoren schwer reduzierbare Oxoverbindungen vorteilhaft zu reduzieren. Insbesondere war es die Aufgabe Phosphinoxide in die entsprechenden Phosphine zu überführen und dabei den Umweg über die korrosiven Dihalogenide und den damit verbundenen Salzanfall zu vermeiden.

Diese Aufgabe wurde erfindungsgemäß gelöst mit einem Verfahren zur reduktiven Deoxigenierung von organischen Verbindungen, die mindestens eine Oxogruppe enthalten unter teilweiser oder vollständiger Bildung der entsprechenden deoxigenierten Verbindungen durch heterogenkatalytische Umsetzung mit Redoxkatalysatoren in ihrer reduzierten oder teilreduzierten Form bei erhöhter Temperatur in der Gasphase, wobei man
a) die Deoxigenierung kontinuierlich im zeitlichen oder räumlichen Wechsel mit der reduzierenden Regenerierung des Redoxkatalysators durchführt und daß man dabei die Reaktion instationär führt, d.h. die zu deoxigenierenden organischen Verbindungen in einer solchen Weise mit dem Redoxkatalysator in Kontakt bringt, daß am Austritt aus der Deoxigenierungszone in seiner Deoxigenierungswirkung noch unverbrauchter Katalysator vorliegt,
   und wobei
b) die Regenerierung des verbrauchten Redoxkatalysators mit gasförmigen Reduktionsmitteln ausgewählt aus der Gruppe bestehend aus Wasserstoff, Kohlenmonoxid, Kohlenwasserstoffen, Ammoniak, Stickstoffmonoxid und Schwefeldioxid bei erhöhter Temperatur durchgeführt wird und
c) ein Redoxkatalysator verwendet wird, der in seiner oxidierten Form mindestens ein reduzierbares Oxid der Metalle ausgewählt aus der Gruppe Bi, V, Cr, Mn, Ti, Fe, Co, Pb, Mo, Ce, U, Sn, Sb und/oder Cu enthält.

In der neuen Reaktion dient der reduzierte Redoxkatalysator sowohl als Katalysator als auch als Sauerstoffakzeptor, wodurch er in eine höhere Oxidationsstufe übergeht und in einer zeitlich oder räumlich getrennten Regenerierung wieder reduziert werden muß, so daß in der Regel mindestens stöchiometrische Mengen des Redoxkatalysators für die Deoxigenierung benötigt werden. In einigen Fällen ist es jedoch auch möglich, die Deoxigenierung in Gegenwart von reduzierenden Gasen durchzuführen, so daß in diesen Fällen geringere als stöchiometrische Mengen des Katalysators erforderlich sind.

Für die technische Realisierung des instationären Reaktionskonzepts gibt es zwei Varianten, nämlich entweder die räumliche oder die zeitliche Trennung der beiden Teilschritte.

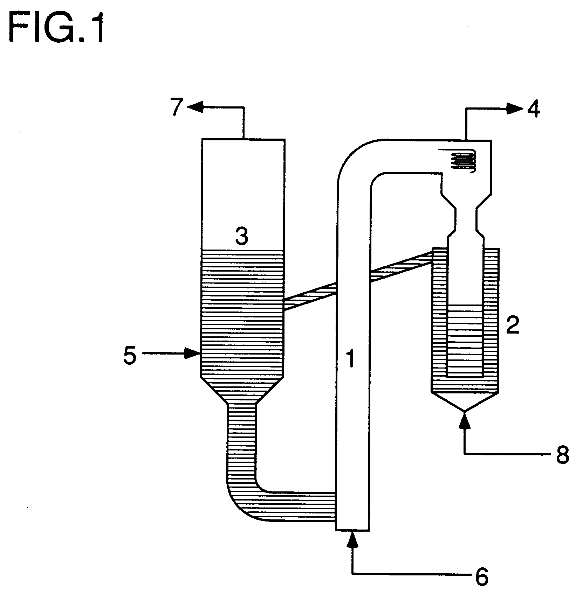

Bei ersterer kommt z.B. ein Wanderbett oder eine zirkulierende Wirbelschicht unter Verwendung eines Riser-Reaktors zur Anwendung, so daß die Katalysatorteilchen aus der Deoxigenierzone, nach Abtrennung der gebildeten Reaktionsprodukte, zu einem separaten Regenerierungsreaktor gefördert werden, in dem die Reduktion des Katalysators durchgeführt wird. Der regenerierte Katalysator wird in die Deoxigenierzone zurückgeführt. Der Prozeß ist kontinuierlich und zyklisch, da der Katalysator fortwährend im Kreis gefördert wird. Der Katalysator ist dabei hohen mechanischen Beanspruchungen ausgesetzt und muß daher über eine ausreichende Härte verfügen. Diese Ausführungsform ist schematisch in Fig. 1 dargestellt. Darin bedeutet 1 den Riser, 2 die Abtrennung des deoxigenierten Produkts 4 vom verbrauchten Katalysator und 3 den Regenerator, wo der verbrauchte Katalysator durch Beaufschlagung mit Reduktionsmittel 5 wieder reduziert wird.

Das zu deoxigenierende Edukt tritt bei 6 ein und das Abgas verläßt bei 7 das System, während bei 8 Inertgas zugeführt wird.

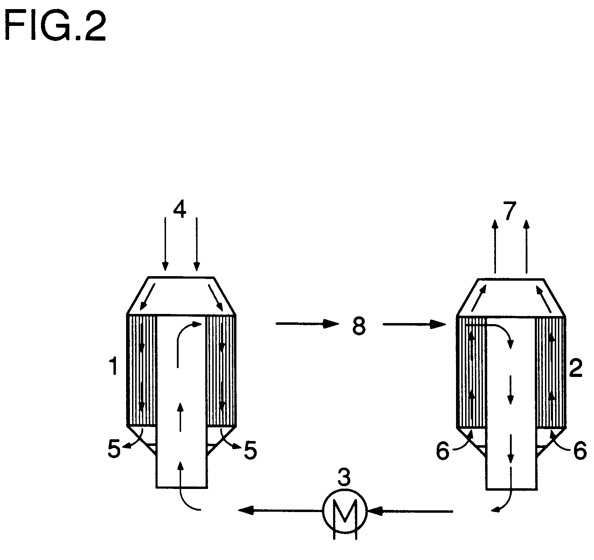

Eine zeitliche Trennung läßt sich in einem Katalysatorfestbett, vorzugsweise einem Rohrreaktor, bei dem im wesentlichen keine Rückvermischung auftritt, so realisieren, daß durch Umschalten der Reaktor periodisch mit der zu deoxigenierenden Verbindung und dem Regeneriergas beschickt wird, wobei auch eine Zwischenspülung mit Inertgas erfolgen kann. Bei Verwendung von mehreren Reaktoren erfolgt die Umschaltung bzw. Weiterschaltung in besonders einfacher Weise, so daß die Deoxigenierungsreaktion und die Regenerierung fortlaufend und parallel erfolgen können. Dies ist in Fig. 2 erläutert. Darin sind Festbettreaktoren, ausgeführt als Rohrbündelreaktoren dargestellt. Während in Reaktor 1 die eigentliche Deoxigenierreaktion abläuft und Produkt erzeugt wird, findet in Reaktor 2 gerade die Regenerierung des verbrauchten Redoxkatalysators mit Reduktionsmittel statt. 3 stellt einen Wärmetauscher dar. Dabei wird das zu deoxigenierende Edukt bei 4 zugeführt und verläßt als oxogruppenfreies Produkt bei 5 den Reaktor. Im Regenerierungsreaktor 2 tritt das Reduktionsmittel (Regeneriergas) bei 6 ein und verläßt als Abgas bei 7 den Reaktor. 8 stellt eine Wärmekopplung dar.

Die instationäre Reaktionsführung, d.h. die Vermeidung einer Gleichgewichtseinstellung durch Aufrechterhaltung eines Reaktionsgradienten - hier des Gradienten der Reduktionsaktivität des Redoxkatalysators unter Vermeidung einer Rückvermischung - wird erreicht, indem eine Rohr-(Pfropfen)strömung eingehalten wird und am Austritt der Deoxigenierungszone immer noch genügende Reduktionsaktivität des Katalysators aufrechterhalten wird.

Der beim erfindungsgemäßen Verfahren zu verwendende Redoxkatalysator besteht in seiner oxidierten Form aus wenigstens einem reduzierbaren Aktivmetalloxid, ausgewählt aus der Gruppe Bi, V, Cr, Mn, Ti, Fe, Co, Pb, Mo, Ce, U, Sn, Cu, das sowohl als reines Oxid bzw. Metall als auch auf einen Träger aufgebracht eingesetzt werden kann. Die Art des Trägers unterliegt im allgemeinen keinen Beschränkungen, sofern er inert ist und die nötige mechanische Festigkeit aufweist. Er ist in der Regel ausgewählt aus der Gruppe Tone, PILC (Pillared Clays), Zeolithe, Aluminiumphosphate, SiC, Si₃N₄, BN, C, und/oder der Metalloxide ausgewählt aus der Gruppe der Oxide des Ti, Zr, Zn, Th, Mg, Ca, Ba, Si, Al. Der Redoxkatalysator kann noch weitere Promotoren, insbesondere (Erd)-Alkali und/oder seltene Erdmetalle enthalten. Trägerkatalysatoren haben den Vorteil einer hohen mechanischen Stabilität im Hinblick auf die fortwährenden Phasen- und Strukturumwandlungen der Aktivkomponente. Die Aktivkomponentenbeladung liegt im Bereich von 2 bis 95 Gew.-%, berechnet als Aktivmetalloxid.

Die Herstellung des Katalysators kann nach sämtlichen bekannten Verfahren erfolgen, z.B. durch Trockenmischen, Imprägnieren, Tränken, Fällung, Cofällung, Sprühtrocknung, Sprühimprägnierung, Eindampfen. Als Katalysatorvorläufer eignen sich die Oxide, Hydroxide, Carbonate, Nitrate, Komplexverbindungen mit anorganischen oder organischen Chelatbildnern, Salze anorganischer und organischer Säuren und organometallische Verbindungen. Diese Katalysatorvorläufer werden durch eine geeignete Hitzebehandlung meist zusammen mit der reduzierenden Aktivierung in die wirksame Katalysatorform überführt.

Es ist einleuchtend, daß eine Aktivität der Systeme bezüglich der Deoxigenierung nur dann vorhanden ist, wenn sich das Aktivmetall nicht in seiner höchsten Oxidationsstufe befindet, sondern wenigstens teilreduziert vorliegt. Im Falle von Bi als Aktivmetall werden die während der Deoxigenierung durch Sauerstoffaufnahme gebildeten Bi-Oxide mit H₂ bei Temperaturen von 300 bis 500°C annähernd bis zum metallischen Bi reduziert. Die aktive Phase zu Reaktionsbeginn ist dann feinverteiltes Bi-Metall auf einem oberflächenreichen Träger. Mit fortschreitender Reaktionsdauer während der Deoxigenierung wird der Katalysator durch Sauerstoffaufnahme zunehmend oxidiert, verliert dadurch an Aktivität und der Umsatz sinkt. Als Folge dieses Desaktivierungsverhaltens durchläuft die Ausbeute an Deoxigenierungsprodukt als Funktion der Reaktionsdauer i.a. ein flaches Maximum oder ein Plateau und fällt dann gleichmäßig ab. In der Technik wird man nicht bis zur vollständigen Desaktivierung des Katalysators warten, sondern die Regenerierung schon vorher einleiten, wenn die Ausbeute oder der Umsatz auf einen bestimmten Wert gefallen sind, wenn z.B. die Ausbeute um 10 bis 20 % unter den Maximalwert gesunken ist. Der bei der jeweiligen Reaktionstemperatur maximal mögliche Oxidationsstufenwechsel des Aktivmetalls (z.B. Bi(O) <-> Bi(III) oder V(III) <-> V(V) bei ≈ 500°C) wird also nicht vollständig durchlaufen, sondern Katalysatornutzungsgrade kleiner als 1 sind zweckmäßig.

Der Katalysator wird im Festbett als stückiger Katalysator eingesetzt, z.B. in Form von Strangpresslingen, Ringen, Ringtabletten, Granulat oder Splitt, Kugeln, Volltabletten oder Netzen mit Abmessungen zwischen 0,5 bis 20 mm.

Für die Verwendung des Katalysators in einem Wanderbett oder Wirbelbett sind dagegen feine Teilchen hoher mechanischer Festigkeit im Größenbereich von 0,01 bis 0,9, vorzugsweise 0,05 bis 0,5 mm angezeigt.

Die Deoxigenierungen nach dem erfindungsgemäßen Verfahren werden bei Reaktionstemperaturen von 100 bis 1000°C, bei Verweilzeiten von 0,01 bis 100 s, bei Drücken von 100 mbar bis 100 bar und mit einer WHSV (Weight Hourly Space Velocity) von 0,01 bis 20 (kg zu deoxigenierende Verbindung/kg Kat.) * h⁻¹ durchgeführt. Neben dem zu deoxigenierenden Edukt können im Feed Verdünnungsmittel wie beispielsweise CO₂, N₂, Edelgase oder Dampf oder auch das Regeneriergas (Reduktionsmittel) selbst zugegen sein. Im speziellen Fall der Reduktion von Phosphinoxiden beträgt die Reaktionstemperatur 400 bis 800°C, die Verweilzeit 0,5 bis 20 s, bei Drücken von 0,5 bis 10 bar und einer WHSV von 0,05 bis 10 h⁻¹.

Die Regenerierung (Reduktion) des verbrauchten, partiell oxidierten Katalysators wird bei Temperaturen im Bereich 100-1000°C mit einem freien Reduktionsmittel, vorzugsweise mit Kohlenwasserstoffen, insbesondere niedermolekularen gesättigten Kohlenwasserstoffen, mit NH₃, NO, SO₂ und besonders bevorzugt mit H₂ und/oder CO, durchgeführt. Auch hier können Verdünnungsmittel im Reaktorzustrom enthalten sein. Die Regenerierung kann bei Unterdruck, Normaldruck oder Überdruck betrieben werden. Bevorzugt sind Drücke im Bereich 100 mbar bis 20 bar.

Auch reaktive Gasmischungen, die das eigentliche Reduktionsmittel erst im Reaktor durch chemische Reaktion freisetzen, sind als Regeneriergase geeignet. So kann beispielsweise der Katalysator zusätzlich mit Cu dotiert werden und als Regeneriergas CO/H₂O verwendet werden. Durch eine in-situ Konvertierungsreaktion am Cu-Zentrum wird dann H₂ gebildet, der das Metalloxid reduzieren kann.

Obgleich das neue Verfahren auf beliebige schwer reduzierbare Oxogruppen enthaltende organische Verbindungen anwendbar ist, eignet es sich vor allem für die Deoxigenerierung von Estern zu Ethern, Nitroverbindungen zu den Nitrosoverbindungen, Oximen oder Aminen, Sulfonsäuren zu Mercaptanen und vor allem von Phosphinoxiden zu Phosphinen oder von Nitrobenzol zu Anilin.

Demgemäß können Phosphinoxide der allgemeinen Formel R¹R²R³P=O zu Phosphinen der allgemeinen Formel R¹R²R³P reduziert werden, wobei R¹, R², R³ unabhängig voneinander für
- einen gegebenenfalls substituierten C₁ bis C₈, vorzugsweise C₁- bis C₆-Alkylrest
- einen gegebenenfalls substituierten C₁ bis C₁₂, vorzugsweise C₁- bis C₈-Cycloalkylrest oder einen gegebenenfalls substituierten Phenylrest der Formel 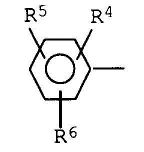
bedeuten, wobei R⁴, R⁵, R⁶ unabhängig voneinander für Wasserstoff, C₁- bis C₄-Alkyl, C₁- bis C₄-Alkoxy, Fluor, Chlor, Brom, COOH oder COOR⁷ stehen, wobei R⁷ C₁- bis C₄-Alkyl bedeutet.

Besonders bevorzugte Ausgangsverbindungen sind
- Trimethylphosphinoxid
- Triethylphosphinoxid
- Tributylphosphinoxid
- Tricyclopentylphosphinoxid
- Tricyclohexylphosphinoxid
- Tritolylphosphinoxid
- Tri-(tert.-butylphenyl)phosphinoxid
- Tri(iso-propylphenyl)phosphinoxid
- Tri-(methoxyphenyl)phosphinoxid
- Tolyldiphenylphosphinoxid
- Phenylditolylphosphinoxid
- Methyldibutylphosphinoxid
und insbesondere Triphenylphosphinoxid.

Eine instationäre Reaktionsführung wurde bereits für zahlreiche Oxidations- und Dehydrierreaktionen beschrieben:

Dabei wird die Oxidations-/Dehydrierreaktion in Abwesenheit von freiem, d.h. dem Eduktstrom kontinuierlich zugesetztem Oxidationsmittel wie molekularem Sauerstoff oder sauerstoffhaltigen Gasen durchgeführt und stattdessen fungiert der aus wenigstens einem reduzierbaren geträgerten Metalloxid bestehende Redoxkatalysator als alleinige Sauerstoffquelle und übernimmt somit die Funktion eines Sauerstoffspeichers. Der oxidische Katalysator wird aufgrund der Abgabe von Gittersauerstoff während der Oxidation/Dehydrierung reduziert und daher im Laufe der Reaktion verbraucht, so daß er in einem zweiten Schritt mit einem Oxidationsmittel, vorzugsweise sauerstoffhaltigen Gasen inklusive reinem Sauerstoff, wieder durch Reoxidation regeneriert werden muß.

Dieses allgemeine Reaktionskonzept der Trennung der beiden Teilschritte von Oxidationsreaktionen unter Einsatz eines reduzierbaren und regenerierbaren Katalysators als echtem Reaktionspartner wurde z.B. schon für die Oxidation bzw. Ammonoxidation von Propen zu Acrolein und Acrylsäure bzw. Acrylnitril beschrieben (GB 885422; GB 999629; K. Aykan, J. Catal. 12 (1968) 281-190), wobei Arsenat- und Molybdatkatalysatoren verwendet wurden. Der Einsatz des Verfahrens bei der oxidativen Dehydrierung von aliphatischen Alkanen zu Mono- und Diolefinen mit Ferritkatalysatoren (z.B. US 3 440 299, DE 2 118 344, DE 1 793 499; sowie US 3 118 007) gehört ebenfalls zum Stand der Technik. Gemäß GB 840 082 nutzt man Fe, Co, Ni-Oxide als feste Sauerstoffträger für die oxidative Dehydrierung von Mono- zu Diolefinen. In zahlreichen Patentschriften wird ebendieses Verfahren für die oxidative Kopplung von Methan zu höheren Kohlenwasserstoffen beschrieben, wobei unterschiedliche Katalysatorklassen zum Einsatz kommen (z.B. US 4 795 849, DE 3 586 769 mit Mn/Mg/Si-Oxiden; US 4 568 789 mit Ru-Oxid; EP 25 44 23 mit Mn/B-Oxiden auf MgO; GB 2 156 842 mit Mn₃O₄-Spinellen;). Auch die Dehydrodimerisierung von Toluol zu Stilben ebenfalls in Abwesenheit von freiem Sauerstoff mittels reduzierbarer Katalysatoren wie Bi/In/Ag-Oxiden (EP 30 837) ist bekannt. Schließlich wird dasselbe Prinzip noch für die Dehydrierung, Dehydrocyclisierung und Dehydroaromatisierung von niederen Alkangemischen zur Benzinveredelung angewandt (US 4 396 537 mit Co/P-Oxid-Katalysatoren sowie US 4 644 089 mit V₂O₅/AlPO). Schließlich werden in JP A2 60/25938 ganz allgemein Oxidations- und Dehydrierreaktionen mit festen oxidischen Sauerstoffträgern beschrieben.

In den Anmeldungen EP 397 637 und EP 403 462 wird das o.g. Verfahrensprinzip für die oxidative Dehydrierung von Aliphaten und Alkylaromaten eingesetzt. Der besonders bevorzugte Redoxkatalysator ist V/MgO. Neuere Patentanmeldungen beinhalten die B₂O₃-Dotierung fester Sauerstoffträger in Form reduzierbarer Metalloxide und deren Anwendung auf die oxidative Kopplung von Methan sowie die oxidative Dehydrierung von Alkanen (EP 254 423), die oxidative Dehydrierung von Alkanen und Alkoholen mit Au/CeO₂(EP 558 148). Die Maleinsäureanhydrid-Synthese aus n-Butan in einem Riser-Regenerator-Reaktor ist in US 4 668 802 erläutert.

Auch in der wissenschaftlichen Literatur sind Abhandlungen über instationäre Reaktionsführung bei Oxidationsreaktionen zu finden: Doroschenko et al., Neftechimia 26 (1986) 48, untersuchen die instationäre oxidative Dehydrierung von Butan mit V-Mg-Mo-O-Systemen. Auch Ogonowski, Chemik XLII 3 (1989) 68, behandelt die Dehydrierung von Butan mit V-Mg-Mo-O/MgO- und Mo-Co-O/MgO-Katalysatoren.

Instationäre Deacon-Verfahren zur Chlorherstellung durch Luftoxidation von HCl werden in DE 40 04 454, US 4 959 202 und WO 91/06505 bzw. US 5 154 911 offenbart.

Alle bisher vorbeschriebene instationäre Verfahren soweit sie zur Durchführung chemischer Redoxreaktionen mit Entkopplung der Reaktionsteilschritte herangezogen werden, betreffen ausnahmslos Oxidations- oder Dehydrierreaktionen. Der Redoxkatalysator dient als Sauerstoffspeicher. Durch Gittersauerstoff wird das Edukt oxidiert. Dabei wird der Sauerstoff in das Eduktmolekül eingebaut oder es wird Wasser gebildet (oxidative Dehydrierungen).

Wie die genannte umfangreiche Literatur über Oxidationsverfahren und die völlige Abwesenheit von entsprechender Literatur über einschlägige Reduktionsverfahren zeigt, lag es offenbar nicht auf der Hand, ein solches Reduktionsverfahren vorzuschlagen, zumal das Bedürfnis für ein geeignetes Verfahren, zumindest für die Reduktion von Phosphinoxiden, seit langem existierte. Demgemäß wurde mit dem neuen Deoxigenierungsverfahren ein Vorurteil überwunden und eine neue Methodik der Deoxigenierung erschlossen.

Ein weiteres technisches Problem, welches mit Hilfe des erfindungsgemäßen Verfahrens überraschend vorteilhaft gelöst werden kann, ist die Deoxigenierung von Nitrobenzol zu Anilin. Auch die instationäre reduktive Deoxigenierung von Nitrobenzol in der Gasphase an vorreduzierten geträgerten Metall(oxid)-Redoxkatalysatoren als regenerierbaren Feststoffreduktantien ist noch nicht bekannt. Vorteilhaft bei dieser Art der Deoxigenierung ist, daß man letztlich mit sehr billigen Reduktionsmitteln, wie CO oder H₂ arbeiten kann, daß man nicht unter hohem Druck arbeiten muß, daß durch die räumliche oder zeitliche Trennung von Reduktionsmittel (CO oder H₂) und Oxidationsmittel (Nitrobenzol) die Arbeitssicherheit viel höher ist und daß durch die sehr hohe Aktivmetallbeladung (vorzugsweise ca. 10 bis 30 Gew.-% Bi) eine gute Resistenz gegen Katalysatorgifte besteht. Für Bi als Katalysatormetall kommt hinzu, daß Bi bei der Reaktionstemperatur flüssig ist und daher immer wieder umgewälzt wird, so daß sich die aktive Katalysatoroberfläche ständig erneuert.

### Beispiel 1

### Katalysatorpräparation:

100 g TiO₂ (Typ DT-51 von Rhone-Poulenc) und 64,6 g basisches Bi-Carbonat Bi₂CO₅ (Merck, enthält 81 Gew.-% Bi) wurden eine Stunde (h) lang trocken gemischt. Die Mischung wurde für 2,5 h im Kneter verdichtet. Während der Knetphase wurde mit 3 Gew.-% organischem Verstrangungshilfsmittel und 107 ml Wasser versetzt. Mittels einer Strangpresse wurde die Knetmasse zu 3 mm Vollsträngen verstrangt. Im Umlufttrockenschrank wurde für 2 h bei 120°C getrocknet und anschließend für 2 h bei 500°C calciniert.

Der Katalysator enthielt 37 Gew.-% Bi₂O₃ und 63 Gew.-% TiO₂. Der Bi/TiO₂-Katalysator hat eine hohe mechanische (Schnitt-) Härte von 33,6 N/Strang und eine BET-Oberfläche von 49,2 m²/g. Für die Deoxigenierung wird eine Splittfraktion von 0,5 - 0,71 mm eingesetzt.

### Deoxigenierungsreaktion und Regenerierung des Katalysators:

Die TPPO-Reduktion wurde in einem Salzbad-Festbettreaktor durchgeführt. Es handelte sich um einen Wendelreaktor mit einem Verhältnis der Rohrlänge zum Rohrinnendurchmesser von 70 cm zu 6 mm, der mit dem bei 500°C mit Wasserstoff vorreduzierten Katalysator beschickt war. Man speiste kontinuierlich TPPO als 6 %ige Lösung in Toluol ein und führte die Reduktion bei 500°C und einer Verweilzeit von 2 s durch. Die Produktgase wurden bei -4°C kondensiert.

Sobald die momentane Ausbeute von TPP 20 % d.Th. unterschritt, wurde mit N₂ gespült und mit Wasserstoff bei 500°C reduziert.

Die Reduktion war nach 1 h beendet und ein neuer Deoxigenierungszyklus schloß sich an.

Man erhielt eine Mischung von 72 % TPP und 28 % TPPO aus der das TPP in an sich bekannter Weise z.B. durch Destillation abgetrennt wurde.

### Beispiel 2

### Katalysatorherstellung

14,57 g Bi(NO₃)₃x5H₂O wurden mit 10 ml konzentrierter HNO₃ und destilliertem Wasser gelöst und dann auf 65 ml Gesamtlösung aufgefüllt. Anschließend wurde die Lösung geteilt und 93 g TiO₂-Träger (4 mm Stränge, Wasseraufnahme = 0,35 ml/g) wurden zweimal mit der Bi-Nitratlösung imprägniert.

Danach wurden die Stränge 16 h bei 120°C getrocknet und 2 h bei 500°C calciniert.

Der Katalysator enthielt 10 Gew.-% Bi₂O₃ und 90 Gew.-% TiO₂. Er hatte eine hohe mechanische (Schnitt)-Härte von 31 N/Strang und eine BET-Oberfläche des fertigen Katalysators von 56,7 m²/g.

### Deoxigenierungsreaktion und Regenerierung des Katalysators:

Die Versuche zur reduktiven Deoxigenierung von Nitrobenzol wurden in einem Wendel-Rohrreaktor mit 20 ml Katalysatorvolumen durchgeführt, der von außen durch eine flüssige Salzschmelze temperiert wurde, so daß annähernd isotherme Bedingungen vorlagen. Vorgelegt wurde eine 0,5 - 0,71 mm-Splittfraktion des Katalysators.

Die Festbett-Katalysatorschüttung wurde zunächst mit H₂ für 1 h bei Reaktionstemperatur vorreduziert und anschließend mit N₂-Gas gespült. Danach wurde eine Mischung aus Nitrobenzol und n-Butanol (als Wasserstoffspender) in N₂-Trägergas über die Schüttung geleitet. Es folgte eine weitere Spülphase mit N₂-Gas. Dieser Zyklus aus Reduktion des Katalysators mit H₂ und der Reduktion des Nitrobenzols wurde ständig wiederholt.

Der in einer Kühlfalle gesammelte integrale Reaktorflüssigaustrag wurde nach jedem Zyklus mittels GC analysiert. Die angewendeten Reaktionsbedingungen, wie die Reaktionstemperatur, die Verweilzeit (VWZ) und die Reaktionszeit sowie die Reaktorergebnisse sind in der folgenden Tabelle dargestellt. Hierbei werden die Reaktionsprodukte in Flächenprozenten angegeben. Die Differenz zu 100 % betrifft Nebenprodukte. Man sieht, daß Anilin mit guten Ausbeuten bei quantitativen Nitrobenzol-Umsätzen erhalten werden kann.

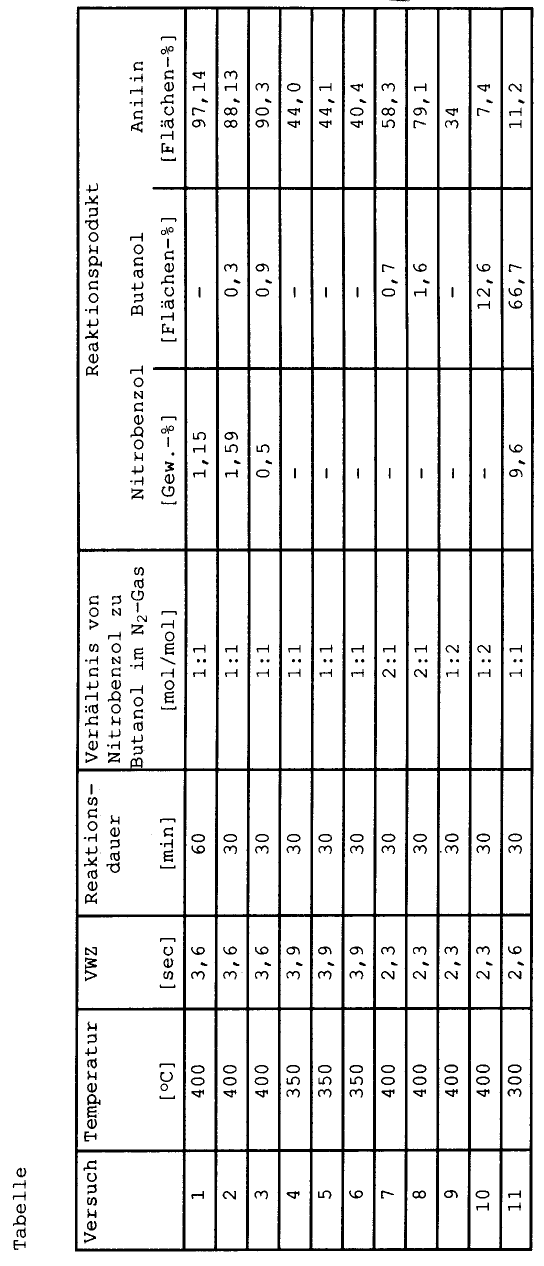

## Patentansprüche

1. Verfahren zur reduktiven Deoxigenierung von organischen Verbindungen, die mindestens eine Oxogruppe enthalten unter teilweiser oder vollständiger Bildung der entsprechenden deoxigenierten Verbindungen durch heterogenkatalytische Umsetzung mit Redoxkatalysatoren in ihrer reduzierten oder teilreduzierten Form bei erhöhter Temperatur in der Gasphase, dadurch gekennzeichnet, daß man
a) die Deoxigenierung kontinuierlich im zeitlichen oder räumlichen Wechsel mit der reduzierenden Regenerierung des Redoxkatalysators durchführt und daß man dabei die Reaktion instationär führt, d.h. die zu deoxigenierenden organischen Verbindungen in einer solchen Weise mit dem Redoxkatalysator in Kontakt bringt, daß am Austritt aus der Deoxigenierungszone in seiner Deoxigenierungswirkung noch unverbrauchter Katalysator vorliegt,
und wobei
b) die Regenerierung des verbrauchten Redoxkatalysators mit gasförmigen Reduktionsmitteln ausgewählt aus der Gruppe bestehend aus Wasserstoff, Kohlenmonoxid, Kohlenwasserstoffen, Ammoniak, Stickstoffmonoxid und Schwefeldioxid bei erhöhter Temperatur durchgeführt wird und
c) ein Redoxkatalysator verwendet wird, der in seiner oxidierten Form mindestens ein reduzierbares Oxid der Metalle ausgewählt aus der Gruppe Bi, V, Cr, Mn, Ti, U, Fe, Co, Pb, Mo, Ce, Sn, Sb und/oder Cu enthält.

2. Verfahren nach Anspruch 1, dadurch gekennzeichnet, daß ein zeitlicher Wechsel der Redoxreaktion dergestalt erfolgt, daß ein Katalysatorfestbett verwendet wird, das durch Umschalten periodisch mit der zu deoxigenierenden Verbindung und dem Regeneriergas beschickt wird.

3. Verfahren nach Anspruch 1, dadurch gekennzeichnet, daß eine räumliche Entkoppelung der Deoxigenierung und der Katalysatorreduktion dergestalt erfolgt, daß bewegliche Katalysatorteilchen unter Verwendung eines Wanderbettes oder einer zirkulierenden Wirbelschicht zwischen einem Deoxigenierreaktor und einem getrennten Regenerierreaktor im Kreis geführt werden.

4. Verfahren gemäß Anspruch 1, dadurch gekennzeichnet, daß man eine Deoxigenierung von Phosphinoxiden zu Phosphinen, Estern zu Ethern, Nitroverbindungen zu den Nitrosoverbindungen, Oximen oder Aminen, oder Sulfonsäuren zu Mercaptanen durchführt.

5. Verfahren gemäß Anspruch 1, dadurch gekennzeichnet, daß man eine Deoxigenierung von Phosphinoxiden der Formel R¹R²R³P=O zu den entsprechenden Phosphinen durchführt, wobei R¹, R² und/oder R³ niedermolekulare Alkyl-, Cycloalkyl- oder Arylreste bedeuten.

6. Verfahren nach Anspruch 1, dadurch gekennzeichnet, daß man Triphenylphosphinoxid, Tricyclohexylphosphinoxid oder Tributylphosphinoxid zu den entsprechenden Phosphinen deoxigeniert.

7. Verfahren nach Anspruch 1, dadurch gekennzeichnet, daß man als Redoxkatalysator einen Trägerkatalysator verwendet, der als aktives Metall Bismut, Titan oder Vanadium in einer Menge von 2 bis 95 Gew.-%, berechnet auf Bi₂O₃, TiO₂ bzw. V₂O₅, enthält.

8. Verfahren gemäß Anspruch 6, dadurch gekennzeichnet, daß man die Deoxigenierungsreaktion bei 400 bis 1000°C und bei 100 mbar bis 100 bar durchführt.

9. Verfahren gemäß Anspruch 1, dadurch gekennzeichnet, daß man Nitrobenzol zu Anilin deoxigeniert.
